Europäisches Patentamt

European Patent Office

Office européen des brevets

(11) Veröffentlichungsnummer : **0 137 278
B1**

(12) **EUROPÄISCHE PATENTSCHRIFT**

(45) Veröffentlichungstag der Patentschrift :
03.01.90

(51) Int. Cl.$^5$ : **A 61 K 47/00**

(21) Anmeldenummer : 84110275.9

(22) Anmeldetag : 29.08.84

(54) Mittel zur transdermalen Applikation von Arzneistoffen.

(30) Priorität : 12.09.83 DE 3333240

(43) Veröffentlichungstag der Anmeldung :
17.04.85 Patentblatt 85/16

(45) Bekanntmachung des Hinweises auf die Patenterteilung : 03.01.90 Patentblatt 90/01

(84) Benannte Vertragsstaaten :
AT BE CH DE FR GB IT LI LU NL SE

(56) Entgegenhaltungen :
DE–A– 2 135 533
DE–A– 2 515 594
GB–A– 1 412 970
GB–A– 2 081 582

(73) Patentinhaber : SCHERING AKTIENGESELLSCHAFT
Berlin und Bergkamen
Müllerstrasse 170/178 Postfach 65 03 11
D-1000 Berlin 65 (DE)

(72) Erfinder : Oloff, Horst, Dr.
Geierpfad 8 c
D-1000 Berlin 28 (DE)
Erfinder : Tack, Johannes-Wilhelm, Dr.
Tharsanderweg 42
D-1000 Berlin 20 (DE)
Erfinder : Windt, Fred
Steglitzer Damm 116a
D-1000 Berlin 41 (DE)
Erfinder : Zimmermann, Ingfried, Dr.
Gollanczstrasse 28c
D-1000 Berlin 28 (DE)

EP 0 137 278 B1

Jouve, 18, rue St-Denis, 75001 Paris, France

## Beschreibung

Die Erfindung betrifft Mittel zur transdermalen Applikation von Arzneistoffen in Form einer Folie, das dadurch gekennzeichnet ist, daß der Arzneistoff zu mindestens 50 % in einem nicht fließfähigen, physiologisch unbedenklichen Gel gelöst ist, welches in einem vernetzten Siliconelastomeren mikrodispers verteilt ist.

Es ist bekannt, daß die Bioverfügbarkeit von oral oder intravenös applizierten Arzneistoffen oft unbefriedigend ist. In den letzten Jahren versuchte man deshalb, die Bioverfügbarkeit der Arzneistoffe dadurch zu verbessern, daß man diese transdermal appliziert. So wird beispielsweise in der DE-A 31 31 610 ein Mittel zur transdermalen Applikation von Glycerintrinitrat beschrieben. Dieses Mittel ist aber nicht geeignet, um feste Arzneistoffe geringer Wasserlöslichkeit transdermal zu applizieren, da die Diffusionsgeschwindigkeit dieser Wirkstoffe in einem derartigen Mittel zu gering ist.

Demgegenüber ist das erfindungsgemäße Mittel sehr gut zur transdermalen Applikation auch von festen Arzneistoffen geringer Wasserlöslichkeit sehr gut geeignet.

Als Arzneistoffe, die im Gel gelöst sind können beispielsweise analgetisch-antirheumatisch wirksame Substanzen mit ulcerogener Nebenwirkung, Antibiotika, Ergot-Verbindungen, ein β-Rezeptorenblocker, ein Prostaglandin, ein Prostacyclin, ein herzwirksames Glykosid, ein pharmakologisch wirksames Peptid oder insbesondere Steroidhormone verwendet werden.

Geeignete Analgetica-Antirheumatica sind beispielsweise Phenylbutazon, Oxyphenylbutazon, Indomethacin, Naproxen, Ibuprofen etc..

Geeignete Antibiotika sind unter anderen die Penicilline, Tetracycline oder Streptomycine.

Geeignete ß-Rezeptorenblocker sind beispielsweise das Pindolol, Mepindolol oder das Propranolol.

Geeignete Prostaglandine oder Prostacycline sind beispielsweise das Iloprost oder das Nileprost.

Geeignete herzwirksame Glykoside sind zum Beispiel die Digitalisglykoside oder die Strophanthoside.

Geeignete pharmakologisch wirksame Peptide sind unter anderem die gonadotropinwirksamen Peptide wie das LHRH.

Geeignete Steroidhormone sind insbesondere die östrogen, gestagen, androgen oder anabol wirksamen Sexualhormone, wie das Östrogen Östradiol und deren Ester, wie das Valerat Benzoat oder Undecylat, Ethinylestradiol etc., Gestagene wie das Norethisteronacetat, Levonorgestrel, Chlormadinonacetat, Cyproteronacetat, Desogestrel oder Gestoden, Androgene wie das Testosteron und seine Ester (Propionat, Undecylat etc.) und Anabolica wie Methandrostenolon, Nandrolon und dessen Ester.

Die Menge des im Mittel enthaltenden Arzneistoffs ist naturgemäß von dessen Wirksamkeit und dessen Resorbierbarkeit abhängig und muß im Einzelfall in üblicher Weise ermittelt werden.

Das Verfahren zur Herstellung dieses Mittels 1, welches sich dadurch auszeichnet, daß man das arzneimittelhaltige Gel in den zur Bildung des vernetzten Siliconelastomeren erforderlichen Komponenten suspendiert und die erhaltene Suspension vulkanisiert, ist ebenfalls Gegenstand der vorliegende Erfindung.

Gut wasserlösliche Arzneistoffe kann man in Gelen lösen, die aus Wasser und einem Verdickungsmittel bestehen. Da das erfindungsgemäße Mittel vorzugsweise zur transdermalen Applikation von in Wasser schwer löslichen Arzneistoffen bestimmt ist, besteht das zur Lösung des Wirkstoffs verwendete Gel vorzugsweise aus einem Verdickungsmittel, einem physiologisch unbedenklichen, hochsiedenden organischem Lösungsmittel und gegebenenfalls Wasser.

Die gegebenenfalls wasserhaltigen Lösungsmittel für die Gelbildung müssen ausreichend lipophil sein um den Arzneistoff zu lösen, andererseits aber auch ausreichend hydrophil sein, um den gewünschten Wirkstofftransport zu ermöglichen. Geeignete Lösungsmittel sind beispielsweise Dimethylformamid oder vorzugsweise mehrwertige Alkohole oder mehrwertige Äther. Als mehrwertige Alkohole oder mehrwertige Äther seien beispielsweise genannt :

Ethylenglykol, Propylenglykol, Glycerin, Ethylenglykolmonomethyläther, Ethylenglykoldimethyläther, Dimethylisosorbid (= 3,6-Dimethoxy-furo [3,2-b]-furan), Diethylenglykol und dessen Äther (« Römpp »*), 814, 815) oder Polyethylenglykole mit einem Molgewicht bis 600 (« Römpp »*), 2750, 2751). Die Hydrophilität der Lösungsmittel kann durch Zugabe von Wasser erhöht werden. Andererseits läßt sich die Lipophilität des Lösungsmittels durch Zugabe unpolarer Öle wie zum Beispiel die unter dem Namen Cetiol[R] bekannten Ester langkettiger Fettsäuren (« Römpp »*), 539) erhöhen.

Das erfindungsgemäße Mittel kann selbstverständlich nur dann wasserfreie Lösungsmittel enthalten, wenn das zur Gelbindung erforderliche Verdickungsmittel in diesem quellfähig ist, wie zum Beispiel Carboxymethylcellulose in Dimethylisosorbit. Ansonsten muß dem Lösungsmittel soviel Wasser zugesetzt werden, daß eine Quellung des Verdickungsmittels möglich ist.

Als Verdickungsmittel (« Römpp »* 3792) werden die in der Galenik zur Gelbildung üblicherweise verwendeten Quellmittel verwendet. Als Verdickungsmittel seien beispielsmäßig genannt ; natürliche organische Verdickungsmittel wie Agar-Agar, Gelatine, Gummi arabicum, Pectine etc., abgewandelte organische Naturstoffe wie Carboxymethylcellulose oder Celluloseäther oder vollsynthetische organische Verdickungsmittel wie Polyacrylverbindungen, Vinylpolymere oder Polyäther. Besonders bevorzugte Verdickungsmittel sind beispielsweise Carbopol[R] der Carboxymethylcellulose.

Die Verdickungsmittel werden so dosiert, daß das entstandene Gel nicht mehr fließfähig ist.

*) Dr. Otto-Albrecht Neumüller: Röpps Chemie Lexikon, 7. Auflage, 1972, Franckh'sche Verlagshandlung Stuttgart, Bundesrepublik Deutschland.

Während das den Arzneistoff enthaltende Gel die innere Phase des erfindungsgemäßen Mittels bildet, besteht die Außenphase des Mittels aus einem vernetzten Siliconelastomeren. Als Siliconelastomeres wird vorzugsweise ein additionsvernetztes RTV-System verwendet, was aber nicht ausschließt, daß auch kondensationsvernetzte RTV-Systeme angewendet werden können. Bevorzugt sind Zweikomponentensysteme (Ullmanns Encyklopedie der technischen Chemie, 4. Auflage, Band 21, (1982), Stichwort « Silicone », Seite 511 ff.). Solche Zweikomponentensysteme bestehen beispielsweise aus :

1-10 Gewichtsprozent Polydimethylhydrogensiloxan mit einem mittleren Molgewicht von 300 bis 2 000

1-10 Gewichtsprozent 2,4,6,8-Tetramethyl-2,4,6,8-tetravinyl-cyclotetrasiloxan 10-100 ppm Platinkatalysator und vinylendengestopptes Polydimethylsiloxan vom mittleren Molgewicht 3 000 bis 20 000.

Es ist üblich zwei Komponenten zu bilden, die erste enthält das Polydimethylhydrogensiloxan und einen Teil des vinylendengestoppten Polydimethylsiloxans.

Die zweite Komponente enthält den Rest des vinylendengestoppten Polydimethylsiloxans das 2,4,6,8-Tetramethyl-2,4,6,8-tetravinylcyclotetrasiloxan und den Katalysator.

Ein geeigneter Platinkatalysator ist beispielsweise das 2,4,6,8-Tetramethyl-2,4,6,8-tetravinyl-cyclotetrasiloxanylplatin.

Durch Variation der Mengenverhältnisse von Polydimethylhydrogensiloxans, vinylendengestopptes Polydimethylsiloxan und 2,4,6,8-Tetramethyl-2,4,6,8-tetravinylsiloxan und Variation der Kettenlänge von Polymethylhydrogensiloxan und vinylendengestopptem Polydimethylsiloxan kann man die Netzgrößenlänge des Siliconelastomeren in weiten Grenzen verändern. Hierdurch gelingt es, erfindungsgemäße Mittel zur transdermalen Applikation von Arzneimittelwirkstoffen mit unterschiedlichsten Diffusionskoeffizienten herzustellen und so jene Mittel bereitzustellen in denen eine optimale Freisetzungsrate des Wirkstoffs erzielt wird.

Der Vernetzungsgrad kann durch Bestimmung der Netzbogenlänge ermittelt werden, indem man das empirisch ermittelte Molekulargewicht zwischen den Vernetzungspunkten (= network chain length) durch das Molekulargewicht einer Siloxaneinheit dividiert. Die empirische Ermittlung des Molekulargewichts zwischen den Vernetzungspunkten kann nach dem in der Publikation von Treloar : The Physics of Rubber Elasticity-Clarendon Press Oxford, 1975, Seite 142 beschriebenen Verfahren erfolgen.

Im allgemeinen ist es sinnvoll, Siliconelastomere mit einer Netzbogenlänge von 20 bis 500 zu wählen, besonders bei Steroidhormonen sind Siliconelastomere mit einer Netzbogenlänge von 50 bis 250 bevorzugt.

Zur Herstellung des erfindungsgemäßen Mittels werden die Komponenten gemischt mit ein Drittel bis drei Gewichtsteilen des den Arzneistoff enthaltenden Gels innig vermischt, so daß die Gelphase mit einer Tröpfchengröße von 5 μm bis 500 μm dispergiert ist, und bis 40 °C bis 100 °C so vulkanisiert, daß Folien von 0,5 bis 5 mm Dicke entstehen.

Der Wirkstofftransport aus dem erfindungsgemäßen Mittel (nachfolgend als « System » bezeichnet) durch seine äußere Grenzfläche in Richtung Haut wird durch den Konzentrationsgradienten Cis/Cas bewirkt (Cis = Konzentration innerhalb, Cas = Konzentration außerhalb des Systems) und läßt sich als Funktion der Grenzfläche A, der Zeit t, der Konzentration Cis und Diffusionskoeffizienten D beschreiben :

$$Q_t = f\ (A,\ t,\ Cis,\ D)$$

Diese makroskopische Betrachtung ist auch gültig für die mikroskopische Betrachtung der Grenzfläche Gel/Silikon im Inneren des Systems. Durch die Grenzfläche A' der inneren Phase zur äußeren Phase diffundiert wegen des Konzentrationsgradienten der Wirkstoff in Richtung der äußeren Begrenzung des Systems.

Der Diffusionskoeffizient D' des Wirkstoffes in der äußeren Phase, die Länge des Diffusionsweges 1 und die Größe der Grenzflächen A' zwischen Innen- und Außenphase bestimmen den Wirkstofftransport innerhalb des Systems :

$$q_t = f\ (A',\ t,\ 1,\ D')$$

Die Größe A' ist abhängig von der Tröpfchenverteilung im System und ist durch Einarbeitungsparameter leicht zu beeinflussen. Durch eine Veränderung des Verhältnisses A'/A über einen weiten Bereich, z. B. 25-500, kann somit die Wirkstoffabgabe des Systems verändert werden.

Aus der allgemeinen Funktionsbeschreibung

$$Q_t = f\ (A'/A,\ t,\ Cis,\ D'/D)$$

wird weiterhin deutlich, daß eine Freigabeänderung durch Variation des Diffusionskoeffizienten D'

3

(= Diffusion innerhalb des Systems) erreichbar ist. Die Veränderung des Diffusionskoeffizienten D' wird erreicht durch gezielten Aufbau der Silikonkautschukphase.

Mit steigendem Vernetzungsgrad sinkt das « Freie Volumen » im Netzwerk und steigt der Diffusionswiderstand.

Die einfache Variabilität der Faktoren A' und D'. führen zu einem bedeutenden Vorteil gegenüber anderen Systemen : Sie stellen voneinander unabhängige Größen zur Einstellung der Wirkstoffabgabe dar.

Zur Herstellung von Pflastern kann man beispielsweise Scheibchen des erfindungsgemäßen Mittels auf eine aluminiumbeschichtete Trägerfolie aufvulkanisieren, diese um das Mittel herum mit einem Adhäsiv (Haftkleber) versehen und mit einer Schutzfolie versiegeln.

Die nachfolgenden Ausführungsbeispiele dienen zur näheren Erläuterung des erfindungsgemäßen Mittels.

## Beispiel 1

A) Bereitung der wirkstoffhaltigen Gel-Phase :

In einer Mischung aus 22,55 g Glycerin 87 %ig mit 22,55 g Dimethyldiglycol und 5,64 g dest. Wasser werden 300 mg Östradiol-17-valerianat gelöst. Die Lösung wird bei Raumtemperatur mit 1,13 g Carbopol[R] 934 unter Rühren verdickt. Zur vollständigen Gelbildung sowie zur Einstellung physiologischer pH-Verhältnisse wird mit ca. 1 n Natronlauge auf pH 5,0 eingestellt.

B) Bereitung der Siliconelastomeren-Phase :

In einem vakuumdicht verschließbaren Reaktionsgefäß mit Rühreinrichtung werden 48,80 g vinylendengestopptes Dimethylpolysiloxan (mittleres Molgewicht 6 000), 0,42 g 2,4,6,8-Tetramethyl-2,4,6,8-tetravinyl-tetracyclosiloxan und 2,99 g Polydimethylhydrogensiloxan mit einem mittleren Molgewicht von 350 bis 500 und 15 ppm Platinkatalysator (in Form einer Toluollösung von 2,4,6,8-Tetramethyl-2,4,6,8-tetravinyl-tetracyclosiloxanylplatin) gemischt.

C) Herstellung des Mittels zur transdermalen Applikation von Östradiol-17-valerianat :

Die gemäß A) hergestellte Gelphase wird der Siliconelastomerenphase zugefügt, das Gemisch unter Vakuum $1,33.10^4$ Pa (100 torr) 20 Minuten lang mit 1 250 Umdrehungen pro Minute gerührt. Dann wird die erhaltene Dispersion in scheibenförmigen Folienformen von 10 cm Oberfläche und 1,5 mm Tiefe bei 60 °C eine Stunde lang vulkanisiert.

## Beispiel 2

A) Zubereitung der wirkstoffhaltigen Gel-Phase :

In einer Mischung aus 8,5 g Dimethyldiglycol, 8,5 g Cetiol[R] HE, 4,0 g dest. Wasser 20,0 g Glycerin 87 %ig, 1,0 g Polyglycol MG 400 und 3,0 ml Pufferlösung pH 9,0 werden 500 mg Testosteron gelöst. Die Lösung wird mit 0,85 g Carbopol[R] 934 unter Rühren verdickt.

B) Bereitung der Siliconelastomeren-Phase :

In einem vakuumdicht verschließbaren Reaktionsgefäß mit Rühreinrichtung werden 51,37 g vinylendengestopptes Dimethylpolysiloxan (mittleres Molgewicht 6 000), 0,93g 2,4,6,8-Tetramethyl-2,4,6,8-tetra-vinyl-tetracyclosiloxan und 0,914 g Polydimethylhydrogensiloxan mit einem mittleren Molgewicht von 350 bis 500 und 15 ppm Platinkatalysator (in Form einer Toluollösung von 2,4,6,8-Tetramethyl-2,4,6,8-tetravinyl-tetracyclosiloxanylplatin) gemischt.

C) Herstellung des Mittels zur transdermalen Applikation von Testosteron :

Die gemäß A) hergestellte Gelphase wird der Siliconelastomerenphase zugefügt, das Gemisch unter Vakuum $1,3.10^4$ Pa (100 torr) 20 Minuten lang mit 1250 Umdrehungen pro Minute gerührt. Dann wird die erhaltene Dispersion in scheibenförmigen Folienformen von 10 cm Oberfläche und 1,5 mm Tiefe bei 60 °C eine Stunde lang vulkanisiert.

## Beispiel 3

A) Zubereitung der wirkstoffhaltigen Gel-Phase :

In einer Mischung aus 10,68 g Glycerin wasserfrei, 49,9 g Cetiol[R] HE, 8,93 g dest. Wasser werden 0,8 g Östradiol gelöst. Diese Lösung wird mit 1,84 g Carbopol(R) 934 unter Rühren verdickt. Zur Verbesserung der Gelstruktur und zur Einstellung physiologischer Bedingungen wird mit ca. 1 n Natronlauge auf pH 5,0 eingestellt.

B) Bereitung der Siliconelastomeren-Phase :

In einem vakuumdicht verschließbaren Reaktionsgefäß mit Rühreinrichtung werden 82,40 g vinylendengestopptes Dimethylpolysiloxan (mittleres Molgewicht 6 000), 0,42 g 2,4,6,8-Tetramethyl-2,4,6,8-tetravinyl-tetracyclosiloxan und 2,99 g Polydimethylhydrogensiloxan mit einem mittleren Molgewicht von 350 bis 500 und 15 ppm Platinkatalysator (in Form einer Toluollösung von 2,4,6,8-Tetramethyl-2,4,6,8-tetravinyl-tetracyclosiloxanylplatin) gemischt.

C) Herstellung des Mittels zur transdermalen Applikation von Östradiol :

Die gemäß A) hergestellte Gelphase wird der Siliconelastomerenphase zugefügt, das Gemisch unter Vakuum $1,3.10^4$ Pa (100 torr) 20 Minuten lang mit 1250 Umdrehungen pro Minute gerührt. Dann wird die erhaltene Dispersion in scheibenförmigen Folienformen von 10 cm Oberfläche und 0,9 mm Tiefe bei 60 °C eine Stunde lang vulkanisiert.

Beispiel 4

A) Zubereitung der wirkstoffhaltigen Gel-Phase :

In einer Mischung aus 5,0 g dest. Wasser, 5,0 g Glycerin wasserfrei und 15,0 g Dimethyldiglycol werden unter leichter Erwärmung (ca. 45 °C) 5,0 g Polyglycol MG 6 000 gelöst.
Nach Abkühlung werden 0,1 g mikrokristalline Lisurid-Base eingearbeitet, es bleiben 30 % des Wirkstoffes ungelöst.
Der Ansatz wird mit 1,0 g Carbopol(R) 934 unter Rühren verdickt und mit ca. 1 n Natronlauge auf pH 5,0 eingestellt.

B) Bereitung der Siliconelastomeren-Phase :

In einem vakuumdicht verschließbaren Reaktionsgefäß mit Rühreinrichtung werden 56,91 g vinylendengestopptes Dimethylpolysiloxan (mittleres Molgewicht 6 000), 0,09 g 2,4,6,8-Tetramethyl-2,4,6,8-tetravinyl-tetracyclosiloxan und 1,04 g Polydimethylhydrogensiloxan mit einem mittleren Molgewicht von 350 bis 500 und 15 ppm Platinkatalysator (in Form einer Toluollösung von 2,4,6,8-Tetramethyl-2,4, 6,8-tetravinyl-tetracyclosiloxanylplatin) gemischt.

C) Herstellung des Mittels zur transdermalen Applikation von Lisurid-Base :

Die gemäß A) hergestellte Gelphase wird der Siliconelastomerenphase zugefügt, das Gemisch unter Vakuum $1,3.10^4$ Pa (100 torr) 20 Minuten lang mit 1250 Umdrehungen pro Minute gerührt. Dann wird die erhaltene Dispersion in scheibenförmigen Folienformen bei 60 °C eine Stunde lang vulkanisiert.

Beispiel 5

A) Zubereitung der wirkstoffhaltigen Gel-Phase :

In 49,52 g Dimethylisosorbid (DMI, Hersteller : Atlas-Chemie, D-4 300 Essen) werden 2,76 g Östradiol gelöst.
Unter Rühren werden 2,72 g Hydroxypropyl-Cellulose (Klucel(R) GF) (Hersteller : Herkules GmbH, D-2 050 Hamburg) eingetragen. Nach 4 Stunden Rühren bei Raumtemperatur resultiert ein Gel.

B) Bereitung der Siliconelastomeren-Phase :

— In einem vakuumdicht verschließbaren Reaktionsgefäß mit Rühreinrichtung werden 47,14 g vinylendengestopptes Dimethylpolysiloxan (mittleres Molgewicht 6 000), 0,55 g 2,4,6,8-Tetramethyl-2,4,6,8-tetravinyl-tetracyclosiloxan und 2,31 g Polydimethylhydrogensiloxan mit einem mittleren Molgewicht von 350 bis 500 und 15 ppm Platinkatalysator (in Form einer Toluollösung von 2,4,6,8-Tetramethyl-2,4,6,8-tetravinyl-tetracyclosiloxanylplatin) gemischt.

C) Herstellung des Mittels zur transdermalen Applikation von Östradiol :

Die gemäß A) hergestellte Gelphase wird der Siliconelastomerenphase zugefügt, das Gemisch unter

5

Vakuum $1,3.10^4$ Pa (100 torr) 20 Minuten lang mit 1250 Umdrehungen pro Minute gerührt. Dann wird die erhaltene Dispersion in scheibenförmigen Folienformen von 10 cm Oberfläche und 0,5 mm Tiefe bei 60 °C eine Stunde lang vulkanisiert.

## Patentansprüche

1. Mittel zur transdermalen Applikation von Arzneistoffen, in Form einer Folie dadurch gekennzeichnet, daß der Wirkstoff zu mindestens 50 % in einem nicht fließfähigen, physiologisch unbedenklichen Gel gelöst ist, welches in einem vernetzten Siliconelastomeren mikrodispers verteilt ist.

2. Mittel gemäß Anspruch 1, dadurch gekennzeichnet, daß der Arzneistoff ein Steroidhormon, eine Ergot-Verbindung, ein β-Rezeptorenblocker, ein Prostaglandin, ein Prostacyclin, ein herzwirksames Glykosid oder ein pharmakologisch wirksames Peptid ist.

3. Mittel gemäß Anspruch 2, dadurch gekennzeichnet, daß der Arzneimittelwirkstoff ein Steroidhormon ist.

4. Mittel gemäß Anspruch 1 bis 3, dadurch gekennzeichnet, daß das Gel eine ein Verdickungsmittel enthaltene, gegebenenfalls wasserhaltige Flüssigkeit eines mehrwertigen Alkohols oder mehrwertigen Äthers ist.

5. Mittel gemäß Anspruch 1 bis 4, dadurch gekennzeichnet, daß das vernetzte Siliconelastomere ein additionsvernetztes Silicon-Kaltelastomeres ist.

6. Mittel nach einem der Ansprüche 1-5 dadurch gekennzeichnet, daß das vernetzte Siliconelastomere eine Netzbogenlänge von 20 bis 500 besitzt.

7. Mittel gemäß Anspruch 6 dadurch gekennzeichnet, daß das vernetzte Siliconelastomere eine Netzbogenlänge von 50 bis 250 besitzt.

8. Verfahren zur Herstellung eines Mittels gemäß Anspruch 1, dadurch gekennzeichnet, daß man das arzneimittelhaltige Gel in den zur Bildung des vernetzten Siliconelastomeren erforderlichen Komponenten suspendiert und die erhaltene Suspension vulkanisiert.

## Claims

1. Agent for the transdermal application of medicinal substances, in the form of a film, characterised in that the active substance is dissolved to at least 50 % in a physiologically acceptable gel that is not capable of flowing and that is micro-dispersed in a cross-linked silicone elastomer.

2. Agent according to claim 1, characterised in that the medicinal substance is a steroid hormone, an ergot compound, a β-receptor blocker, a prostaglandin, a prostacyclin, a cardio-active glycoside or a pharmacologically active peptide.

3. Agent according to claim 2, characterised in that the medicinal active substance is a steroid hormone.

4. Agent according to claims 1 to 3, characterised in that the gel is a liquid of a polyhydric alcohol or polyvalent ether which contains a thickening agent and, optionally, water.

5. Agent according to claims 1 to 4, characterised in that the cross-linked silicone elastomer is a cold silicone elastomer cross-linked by addition.

6. Agent according to any one of claims 1 to 5, characterised in that the cross-linked silicone elastomer has a network curve length of from 20 to 500.

7. Agent according to claim 6, characterised in that the cross-linked silicone elastomer has a network curve length of from 50 to 250.

8. Process for the manufacture of an agent according to claim 1, characterised in that the gel containing the medicament is suspended in the components required for forming the cross-linked silicone elastomer and the resulting suspension is vulcanised.

## Revendications

1. Produit pour application transdermique de médicaments, ce produit étant sous forme d'une feuille et étant caractérisé en ce que la substance active est dissoute à 50 % au moins dans un gel non fluide, ne soulevant pas d'objection d'ordre physiologique et qui est réparti en microdispersion dans un élastomère de silicone réticulée.

2. Produit selon la revendication 1, caractérisé en ce que le médicament est une hormone stéroïdienne, un composé du type ergot (de seigle), un agent de blocage des β-récepteurs, une prostaglandine, une prostacycline, un glycoside pouvant agir sur le cœur ou un peptide doué d'une activité pharmacologique.

3. Produit selon la revendication 2, caractérisé en ce que le médicament est une hormone stéroïdienne.

4. Produit selon l'une des revendications 1 à 3, caractérisé en ce que le gel est un liquide

EP 0 137 278 B1

éventuellement aqueux, formé d'un polyalcool ou d'un poly-étheroxyde et contenant un épaississant.

5. Produit selon l'une quelconque des revendications 1 à 4, caractérisé en ce que l'élastomère de silicone réticulée est un élastomère de silicone durcie ou vulcanisée à froid et réticulée par addition.

6. Produit selon l'une quelconque des revendications 1 à 5, caractérisé en ce que l'élastomère de silicone réticulée possède une longueur de maille de réseau de 20 à 500.

7. Produit selon la revendication 6, caractérisé en ce que l'élastomère de silicone réticulée possède une longueur de maille de réseau de 50 à 250.

8. Procédé de préparation d'un produit selon la revendication 1, caractérisé en ce qu'on met le gel contenant le médicament en suspension dans les composants nécessaires pour la formation de l'élastomère de silicone réticulée et l'on soumet la suspension ainsi obtenue à un durcissement ou une « vulcanisation ».